# DEMANDE DE BREVET EUROPEEN

(11) **EP 2 116 208 A1**
(43) Date de publication de la demande: **11.11.2009**
(21) Numéro de dépôt: 09290315.2
(22) Date de dépôt: 30.04.2009
(51) Int. Cl.: A61B 19/00, A61B 17/16

(54) **Instrument chirurgical invasif muni d'un transpondeur**

(30) Priorité: 06.05.2008 FR 0802521
(71) Demandeur: SFERIC STELLITE, 41500 Menars (FR)
(72) Inventeur: Grimard, Jean-Christophe, 41120 Cellettes (FR)
(74) Mandataire: Debay, Yves

(57) **Abrégé**

La présente invention concerne un système de lecture (4) et son utilisation destiné à coopérer avec un transpondeur (2) disposé sur un instrument chirurgical de façon à assurer rapidement l'identification de l'instrument chirurgical par approchement du système de lecture (4) au transpondeur (2) de l'instrument chirurgical puis lecture des informations associées à l'instrument contenues dans le transpondeur (2) et communication avec un système informatique (5), **caractérisé en ce que** le transpondeur (2) est enfermé dans un logement (13) formé dans l'instrument destiné à recevoir à l'intérieur le transpondeur (2), ledit logement (13) étant refermé de façon étanche par un couvercle fin (14), de même manière que l'instrument chirurgical, soudé ou collé (3) au corps (10) de l'instrument chirurgical de façon à ce que la surface externe de l'instrument chirurgical ne présente aucune aspérité saillante, le transpondeur (2) communiquant ou enregistrant par le système de lecture (4) des informations concernant l'historique.

## Description

La présente invention concerne le domaine de l'instrumentation chirurgicale. Elle concerne, plus particulièrement, un instrument chirurgical invasif, tel qu'une râpe fémorale, muni d'un transpondeur.

Les instruments médicaux, et plus particulièrement, les instruments chirurgicaux, font généralement l'objet d'un suivi rigoureux destiné à garantir leurs conditions d'utilisation de décontamination et la sécurité des patients. Ce suivi consiste à enregistrer et mettre à jour toute information utile portant sur l'instrument, par exemple, son numéro de série, les dates de ses opérations de maintenance ou de stérilisation, le nombre d'utilisations, etc. A cet effet, les étiquettes électroniques, encore appelées transpondeurs ou TAGs RFID (en anglais, radio frequency identification), sont largement utilisées car elles présentent des avantages considérables. Solidaires d'un instrument, elles permettent, en association avec un module de lecture et/ou écriture doté d'une antenne et un ordinateur central, l'écriture, la lecture et le stockage d'un grand nombre de données relatives à cet instrument. L'enregistrement d'une information peut être réalisé automatiquement, lors d'une étape de nettoyage, par exemple, de façon à limiter l'erreur d'origine humaine. Un tel mode de fonctionnement est décrit, par exemple, dans le document EP 0 992 212. Les étiquettes électroniques sont, en outre, extrêmement fiables et supportent les températures élevées des procédés de stérilisation.

Concernant les instruments métalliques simples, tels que des ciseaux, des scalpels, etc., les transpondeurs sont généralement fixés sur une zone non fonctionnelle de l'outil, par exemple, son manche. Ils sont alors disposés dans une boîte métallique, elle même soudée au manche.

Pour les instruments plus complexes comportant plusieurs pièces montées ensemble dont au moins un outil, le transpondeur est généralement solidaire d'une pièce extérieure de façon à être facilement accessible en lecture et en écriture. Le document EP 1 480 153 montre, par exemple, un endoscope formé d'une partie invasive, d'une unité de couplage optique et d'une commande munie d'un transpondeur. Cette disposition du transpondeur sur une pièce externe de l'endoscope n'est pas idéale, particulièrement en cas de démontage partiel ou total de l'instrument, pour un nettoyage, par exemple, ou pour le changement d'un élément. En effet, une fois l'instrument démonté, les différentes pièces et en particulier, les pièces actives ne sont plus identifiables. Si plusieurs instruments sont démontés simultanément, leurs pièces peuvent être interverties au remontage. Par conséquent, seule la traçabilité de la pièce étiquetée peut être réellement garantie. Plus grave même, une incompatibilité entre les différentes pièces peut survenir suite à une telle interversion. Le fonctionnement de l'instrument et, par conséquent, la santé du patient, sont alors hypothéqués.

Il est également connu, par le document EP 1 774 917, un instrument médical comportant un élément tubulaire métallique, un outil fixé à l'une de ses extrémités et des moyens d'actionnement de l'outil couplés à son autre extrémité. Selon ce document, l'élément tubulaire renferme un transpondeur passif cylindrique contenant des informations relatives à l'outil. Mais ce document ne concerne qu'un certain type d'instrument médical, tel qu'un laparoscope, comportant un outil interchangeable solidaire d'une tige montée à l'intérieur d'un actionneur.

Enfin, le document FR 2 811 923 propose une râpe destinée à préparer le canal intra-médullaire d'un os pour que celui-ci reçoive ultérieurement une prothèse. La râpe comprend un corps, allongé selon un axe sensiblement parallèle à l'axe longitudinal du canal, à section transversale variable, constitué d'une face dite supérieure et d'une enveloppe extérieure et ayant une forme apte à collaborer avec la paroi interne du canal intra-médullaire de l'os. La râpe se termine par une forme profilé pour faciliter l'enfoncement de la râpe dans le canal. Des éléments géométriques mâles ou femelles, disposés sur la face supérieure du corps constituent un système d'encliquetage destiné à recevoir un embout constituant l'extrémité d'un manche pour manipuler la râpe dans le canal et destiné à recevoir éventuellement des composants prothétiques d'essais.

La présente invention a pour but de pallier un ou plusieurs des inconvénients de l'art antérieur et propose un instrument chirurgical invasif, tel qu'une râpe fémorale, formant une pièce monobloc dont la traçabilité peut être assurée facilement par un système de lecture.

Pour atteindre ce but, le système de lecture destiné à coopérer avec un transpondeur disposé sur un instrument chirurgical de façon à assurer rapidement l'identification de l'instrument chirurgical par approchement du système de lecture au transpondeur de l'instrument chirurgical puis lecture des informations associées à l'instrument chirurgical contenues dans le transpondeur et communication avec un système informatique **caractérisé en ce que** le transpondeur est enfermé dans un logement formé dans l'instrument chirurgical destiné à recevoir à l'intérieur le transpondeur, ledit logement étant refermé de façon étanche par un couvercle fin, de même matière que l'instrument chirurgical, soudé ou collé au corps de l'instrument chirurgical de façon à ce que la surface externe de l'instrument chirurgical ne présent aucune aspérité saillante, le transpondeur communiquant ou enregistrant par le système de lecture des informations concernant l'historique, les positions de l'instrument chirurgical dans une boîte étant affichées sur un écran du système informatique par affichage des données provenant d'une base de données et associées à la traçabilité.

Selon une autre particularité, l'instrument chirurgical invasif forme une pièce monobloc comprenant le logement formé par une opération d'usinage du corps de l'instrument chirurgical dans une zone lisse ou en retrait du corps qui ne fragilise pas l'instrument chirurgical et ne gêne pas la mise en place d'une poignée.

Selon une autre particularité, les dimensions du logement et l'épaisseur réduite du couvercle permettent de positionner le transpondeur près de la surface externe du corps de l'instrument chirurgical.

Selon une autre particularité, le logement et le couvercle sont de forme sensiblement cylindrique.

Selon une autre particularité, le couvercle du logement de l'instrument chirurgical est réalisé en acier inoxydable ou en titane ou en chrome cobalt ou en polymère ou tous autres matériaux présentant des caractéristiques de résistance à la corrosion et de résistance mécanique, le même matériau étant utilisé pour former le corps de l'instrument chirurgical.

Selon une autre particularité, le couvercle est assemblé par soudage, tel le soudage par laser, de façon à assurer une continuité métallurgique entre la surface externe du corps de l'instrument chirurgical et le couvercle et assurer l'étanchéité du logement.

Selon une autre particularité, le couvercle est collé au corps de l'instrument chirurgical de façon à assurer l'étanchéité du logement.

Selon une autre particularité, un système de lecture est destiné à coopérer avec le transpondeur enfermé et calé dans le logement de l'instrument chirurgical de façon à assurer rapidement la traçabilité du transpondeur par approchement du système de lecture vers le couvercle du logement de l'instrument chirurgical puis lecture des informations caractéristiques de l'instrument chirurgical contenues dans le transpondeur qui s'affichent, après transmission du système de lecture vers un système informatique, sur un écran du système informatique.

Selon une autre particularité, l'instrument chirurgical est une râpe fémorale comportant un corps allongé, parallèle à l'axe longitudinal du canal de l'os à opérer et une partie supérieure destinée à être utilisée comme moyen de préhension pour manipuler la râpe fémorale, le corps de la râpe fémorale comportant une zone lisse supérieure et se terminant par une forme profilé en fuseau pour faciliter l'enfoncement de la râpe fémorale dans le canal de l'os, la forme profilé de la râpe fémorale comportant une denture pour permettre de râper le canal de l'os.

Selon une autre particularité, le système de lecture est apte à identifier l'utilisateur de l'instrument chirurgical à l'aide d'un badge.

Selon une autre particularité, le système informatique est un ordinateur personnel portable.

Un autre but est atteint en proposant un procédé d'intégration d'un transpondeur dans un instrument chirurgical selon l'invention, **caractérisé en ce qu**'il comprend :
- une étape d'usinage ou d'alésage du corps de l'instrument chirurgical pour former le logement puis la cavité destiné à recevoir le couvercle du logement de l'instrument chirurgical ;
- une étape de mise en place du transpondeur dans le logement précédemment usiné de l'instrument chirurgical ;
- une étape de mise en place du couvercle dans la cavité précédemment usinée du corps de l'instrument chirurgical ; et
- une étape de soudage ou de collage du couvercle fin au corps de l'instrument chirurgical de façon à ce que la surface externe de l'instrument chirurgical ne présente aucune aspérité saillante.

Selon une autre particularité, les dimensions du logement et l'épaisseur réduite du couvercle permettent de positionner le transpondeur près de la surface externe du corps de l'instrument chirurgical.

Selon une autre particularité, les alésages pour former le logement et la cavité prévue pour recevoir le couvercle sont réalisés selon un axe perpendiculaire à la surface externe du corps de l'instrument chirurgical.

Selon une autre particularité, le diamètre de l'alésage formant le logement est inférieur au diamètre de l'alésage formant la cavité pour le couvercle et la profondeur de l'alésage formant le logement est supérieure à la profondeur de l'alésage formant la cavité pour le couvercle.

Selon une autre particularité, le diamètre et la profondeur du logement formé par usinage sont respectivement sensiblement supérieurs au diamètre et à la profondeur du transpondeur de façon à ce que le transpondeur soit calé dans le logement lorsque le couvercle est soudé ou collé au corps de l'instrument chirurgical.

Un autre but est atteint en proposant une utilisation d'un instrument chirurgical selon l'invention, **caractérisée en ce que** l'instrument chirurgical est destiné à coopérer avec un système de lecture relié à un système informatique gérant une base de données, l'utilisation comportant :
- une étape de lecture des informations contenues dans le transpondeur calé dans le logement de l'instrument chirurgical au moyen du système de lecture ;
- une étape de transmission des informations lues par le système de lecture à destination du système informatique ; les informations pouvant être éventuellement transmises par internet au fabricant et/ou établissement où est livré l'instrument chirurgical.
- une étape de recherche, au moyen d'un logiciel approprié du système informatique, dans la base de données du système informatique des données de traçabilité associées à l'instrument chirurgical ; et
- une étape d'affichage sur l'écran (50) du système informatique (5) de l'emplacement pour l'instrument chirurgical dans la boîte de rangement et des données de traçabilité associées à l'instrument chirurgical telles que le numéro de boîte de rangement, le numéro du logement de l'instrument chirurgical dans ladite boîte, le numéro de l'instrument chirurgical et l'historique clinique associé à l'instrument chirurgical ou les données relatives aux patients ou les données opératoires des interventions chirurgicales précédentes.

Selon une autre particularité, les données informatiques associées à l'instrument chirurgical et affichées sur l'interface du système informatique peuvent être sélectionnées par l'utilisateur via une interface utilisateur du système informatique et peuvent renseignées sur les conditions d'utilisation, de nettoyage, de stérilisation et de stockage de l'instrument chirurgical.

Selon une autre particularité, les données de traçabilité associées à l'instrument chirurgical sont aptes à être modifiées au fur et à mesure des utilisations de l'instrument chirurgical, les modifications étant saisies au moyen de l'interface du système informatique puis retransmises via le système de lecture au transpondeur de façon à ce qu'elles soient mémorisées dans le transpondeur.

Selon une autre particularité, les données de traçabilité associées à l'instrument chirurgical peuvent être modifiées au fur et à mesure des utilisations de l'instrument chirurgical, les modifications étant saisies au moyen de l'interface utilisateur du système informatique puis retransmises via le système de lecture au transpondeur de façon à ce qu'elles soient mémorisées dans la mémoire du transpondeur.

Selon une autre particularité, les données de traçabilité associées à l'instrument chirurgical, une fois transmises au système informatique, peuvent être transmises par internet au fabricant et/ou à l'établissement où est livré l'instrument chirurgical.

Selon une autre particularité, une alarme sonore du système informatique informe de l'usure de l'instrument chirurgical et de la prévision du rachat d'un nouvel instrument chirurgical.

L'invention sera mieux comprise et d'autres buts, caractéristiques, détails et avantages de celle-ci apparaîtront plus clairement au cours de la description explicative qui va suivre faite en référence aux figures annexées données à titre d'exemples non limitatifs dans lesquelles :
- la figure 1 représente une vue en coupe d'une partie du corps d'un instrument chirurgical, tel qu'une râpe fémorale, pourvu d'un transpondeur disposé dans un logement usiné ;
- la figure 2 représente une vue en coupe d'une partie du corps d'un instrument chirurgical, tel qu'une râpe fémorale, dans lequel un logement a été usiné pour recevoir un transpondeur ;
- la figure 3 représente une vue en coupe d'une râpe fémorale pourvue d'un logement destiné à recevoir un transpondeur ;
- la figure 4 illustre le procédé d'intégration d'un transpondeur dans un instrument chirurgical formé d'une pièce monobloc ; et
- la figure 5 illustre l'utilisation de l'instrument chirurgical coopérant avec un système de lecture associé à un système informatique.

L'invention concerne un instrument chirurgical formant une pièce monobloc, tel qu'une râpe fémorale (1), équipé d'un transpondeur (2).

La suite de la description fait référence à une râpe fémorale (1) pour les prothèses de hanches mais l'invention s'applique à tout instrument chirurgical formant une pièce monobloc.

De façon avantageuse, la râpe fémorale (1) équipée d'un tel transpondeur (2) permet d'améliorer son suivi ou traçabilité entre les différents établissements de santé et sa gestion, comme par exemple, l'utilisation sur tels patients, la gestion des stocks, etc.

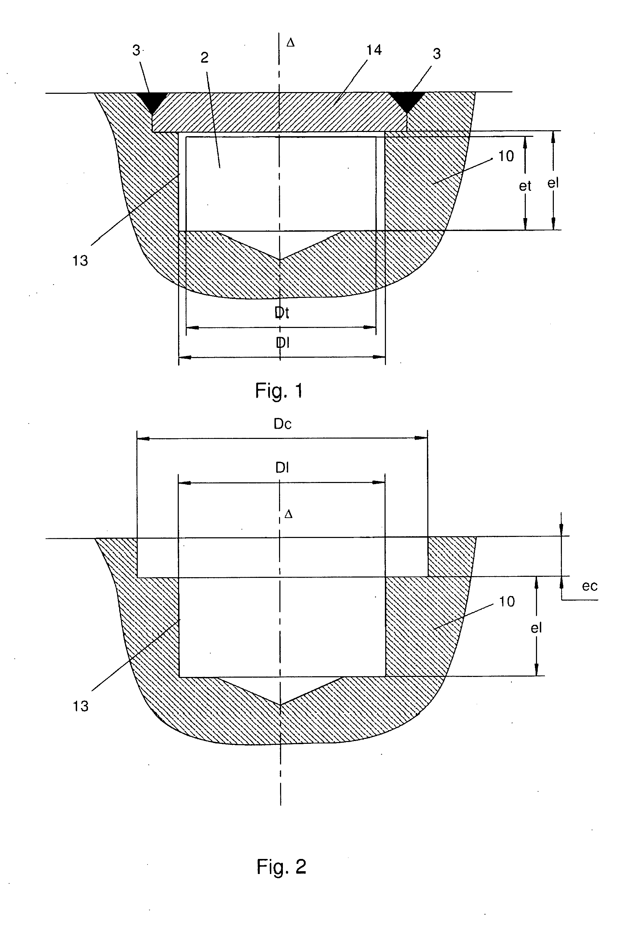

La râpe fémorale (1) représentée sur les figures 1 à 3, comporte classiquement un corps (10) allongé, parallèle à l'axe longitudinal du canal de l'os à opérer. La râpe fémorale (1) comprend une partie supérieure (11) destinée à être utilisée comme moyen de préhension pour manipuler la râpe fémorale (1). Le corps (10) de la râpe fémorale (1) comporte une zone supérieure lisse (12a) et se termine par une forme profilé (12b) en fuseau pour faciliter l'enfoncement de la râpe fémorale (1) dans le canal de l'os. La forme profilée (12b) du corps (10) de la râpe fémorale (1) comporte une denture pour permettre de râper le canal de l'os. Il est à noter que la râpe fémorale (1) forme une pièce monobloc.

En outre, la râpe fémorale (1) présente la caractéristique d'être réalisée en matériau inoxydable comme par exemple, un acier inoxydable ou un titane TA6V ou un chrome cobalt ou en polymère.

La forme géométrique de la râpe fémorale (1) est particulière et dépend de la prothèse ou implant à insérer dans le canal de l'os préalablement râper au moyen de la râpe fémorale (1).

La râpe fémorale (1) comprend un logement (13) formé par une opération d'usinage ou d'alésage du corps (10) de la râpe fémorale (1) dans la zone supérieure lisse (12a) ou dans une zone en retrait par rapport à la denture de la forme profilé (12b) du corps (10) de la râpe fémorale (1) de façon à ne pas fragiliser la râpe fémorale (1).

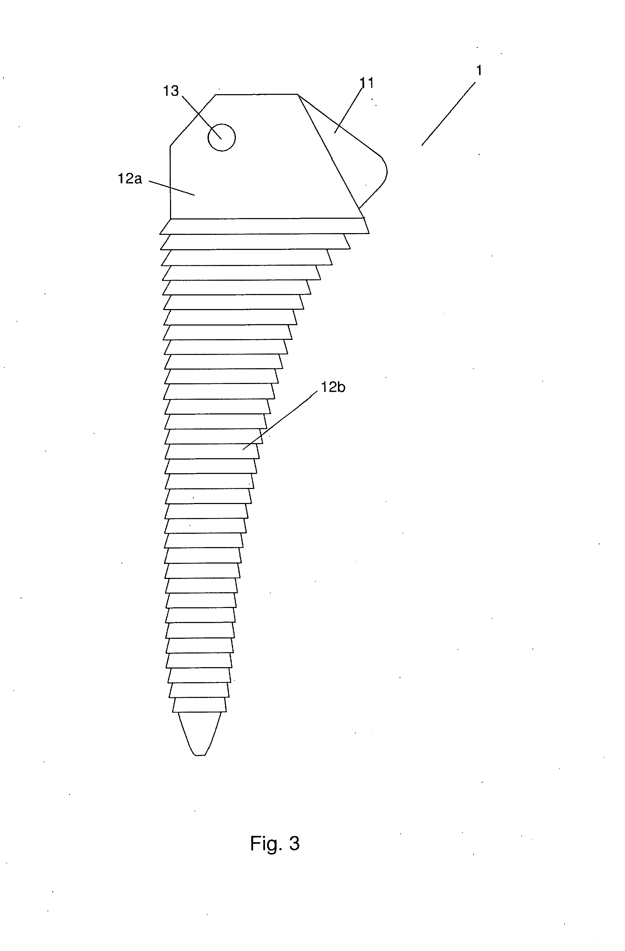

Dans l'exemple représenté à la figure 3, le logement (13) est prévu dans la zone supérieure lisse (12b) du corps (10) de la râpe fémorale (1).

Le logement (13), de forme sensiblement cylindrique et débouchant sur la surface externe lisse de la râpe (1), est destiné à recevoir à l'intérieur un transpondeur (2) ou une étiquette électronique ou encore un TAG RFID puis est refermé de façon étanche par un couvercle fin (14) soudé ou collé (3) au corps (10) de la râpe fémorale (1) de façon à ce que la surface externe de la râpe fémorale (1) ne présente aucune aspérité saillante.

En effet, la râpe fémorale (1) étant un instrument chirurgical invasif, il est absolument nécessaire que celle-ci reprenne sa forme initiale c'est-à-dire avant l'usinage du logement (13) dans le corps (10).

De manière non limitative, le couvercle (14), de forme sensiblement cylindrique, du logement (13) de la râpe (1) est réalisé en acier inoxydable ou en titane ou en chrome cobalt ou en polymère ou tous autres matériaux présentant des caractéristiques de résistance à la corrosion et de résistance mécanique.

De façon avantageuse, l'épaisseur réduite du couvercle (14) du couvercle et les dimensions du logement (13) permettent de positionner le transpondeur (2) sensiblement à proximité de la surface externe du corps (10) de la râpe fémorale (1) de façon à ce que les informations contenues dans le transpondeur (2) puissent être lues aisément.

Il est à noter que le même matériau sera utilisé pour former le corps (10) de la râpe (1) et le couvercle (14) du logement (13) de la râpe (1).

De manière non limitative, l'opération de soudage (3) du couvercle (14) au corps (10) de la râpe (1) est réalisée, sans affecter le fonctionnement du transpondeur (2), par laser de façon à assurer une continuité métallurgique entre la surface externe du corps (10) de la râpe (1) et le couvercle (14) ou par tous autres types de soudage permettant d'assurer cette continuité métallurgique.

De façon avantageuse, l'opération de soudage ou de collage (3) permet d'assurer l'étanchéité du logement (13) de la râpe fémorale (1) de façon à ne pas affecter le transpondeur (2) et les informations qu'il contient lors, par exemple, des opérations de stérilisation de la râpe fémorale (1) ou tous autres opérations de nettoyage ou d'utilisation de la râpe fémorale (1), etc. En effet, le transpondeur (2) résiste aux opérations de stérilisation réalisées à de hautes températures, par exemple 135°C, mais ne résiste pas à l'humidité. Il est, par conséquent, nécessaire que le logement (13) formé dans le corps (10) de la râpe fémorale (1) soit étanche pour protéger le transpondeur (2) de l'humidité.

Il est à noter que le transpondeur (2) peut supporter des températures maximales de l'ordre de 250°C.

Il est également à noter que seulement un transpondeur (2) est inséré dans le logement (13) formé dans la râpe fémorale (1).

De façon avantageuse, le transpondeur (2) ainsi inséré dans son logement (13) est protégé des chocs mécaniques au cours de l'utilisation et du nettoyage de la râpe fémorale (1).

Les transpondeurs (2) passifs cylindriques sont connus de l'homme du métier. Ils sont employés pour le marquage d'objets. Les transpondeurs (2) comportent un circuit électronique muni de deux plages de contact et une bobine formée d'un fil enroulé sur un noyau en ferrite et dont les extrémités sont soudées aux plages de contact. La lecture/écriture de ces transpondeurs (2) est réalisée à l'aide de l'antenne formée du bobinage annulaire apte à émettre et recevoir un signal radiofréquence (RF) contenant des informations (numéro de série, dates des opérations de maintenance ou de stérilisation, etc.) et d'un ordinateur central destiné à stocker et traiter l'information issue du signal RF.

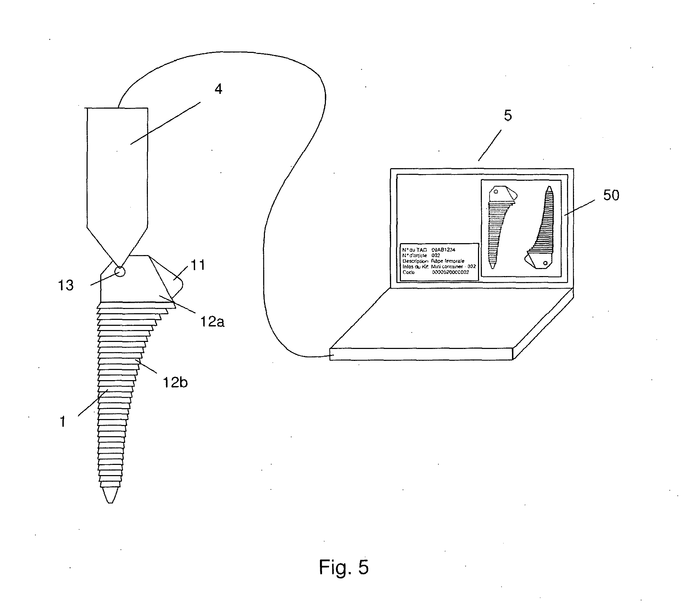

En référence à la figure 5, la présente invention propose également une utilisation de l'instrument chirurgical, tel qu'une râpe fémorale (1), muni d'un transpondeur (2), l'instrument chirurgical étant destiné à coopérer avec un système de lecture (4) relié à un système informatique (5).

L'utilisation de l'instrument chirurgical comporte :
- une étape de lecture des informations contenues dans le transpondeur (2) calé dans le logement (13) de l'instrument chirurgical au moyen du système de lecture (4) ;
- une étape de transmission des informations lues par le système de lecture (4) à destination du système informatique (5) ;
- une étape de recherche dans la base de données, au moyen d'un logiciel approprié du système informatique (5), des données de traçabilité associées à l'instrument chirurgical ; et
- une étape d'affichage sur l'interface (50) du système informatique (5) de l'emplacement pour l'instrument chirurgical dans la boîte de rangement et des données de traçabilité associées à l'instrument chirurgical.

L'étape d'affichage permet d'accéder aux données de traçabilité liées à l'instrument chirurgical telles que le numéro de boîte de rangement, le numéro du logement de l'instrument chirurgical dans ladite boîte, le numéro de l'instrument chirurgical, l'historique clinique associé à l'instrument chirurgical, les données relatives aux patients et les données opératoires des interventions chirurgicales précédentes.

Les données informatiques associées à l'instrument chirurgical et affichées sur l'interface (50) du système informatique (5) peuvent être sélectionnées par l'utilisateur via une interface utilisateur telle qu'un clavier ou une souris reliée au système informatique (5) et peuvent renseignées sur les conditions d'utilisation, de nettoyage, de stérilisation et de stockage de l'instrument chirurgical. Ces données informatiques peuvent être stockées dans le système lui-même ou être reliées à un serveur à distance par une connexion à internet, par exemple. Leur mise à jour peut ainsi être gérée à distance.

L'étape de lecture du transpondeur (2) de l'instrument chirurgical consiste soit à approcher le système de lecture (4) qui par un capteur électronique à courte distance, environ 2 à 3 millimètres du couvercle (14) du logement (13) de la râpe fémorale (1), permet de lire les informations contenues dans le transpondeur (2) et associées à l'instrument chirurgical soit à relier le système de lecture (4) au transpondeur (2) au moyen d'une sonde de lecture qui permet de lire les informations contenues dans le transpondeur (2) et associées à l'instrument chirurgical. Les informations lues par le système de lecture (4) sont envoyées au système informatique (5) qui par un logiciel approprié effectue l'étape de recherche dans la base de données. Puis, ces informations ou données de traçabilité associées à l'instrument chirurgical peuvent éventuellement être avantageusement transmises par internet au fabricant et/ou à l'établissement où est livré l'instrument chirurgical, cet échange faisant office de fiche navette.

En outre, les données de traçabilité associées à l'instrument chirurgical peuvent être modifiées au fur et à mesure des utilisations de l'instrument chirurgical, par exemple, les données liées à la dernière intervention chirurgicale telles que le nom du dernier patient, le nom du chirurgien, le nom de l'établissement de santé. Ces modifications sont saisies au moyen de l'interface utilisateur, par exemple via le clavier, du système informatique (5) puis retransmises via le système de lecture (4) au transpondeur (2) de façon à ce qu'elles soient mémorisées dans la mémoire du transpondeur (2). De façon avantageuse, les données stockées dans la mémoire du transpondeur (2) sont réinscriptibles.

La gestion de l'instrument chirurgical peut donc se faire par le service responsable d'un hôpital ou une société compétente éloignée.

Le système informatique (5) est, par exemple, un ordinateur personnel comportant au moins des moyens d'accès à un serveur via un réseau de communication et une interface utilisateur.

Avantageusement, l'ensemble du système de lecture et système informatique peut être un ordinateur personnel portable. Il peut être installé et utilisé directement en bloc opératoire ou dans des salles annexes de préparation, contrôle, nettoyage, stérilisation, conditionnement des instruments. Les opérateurs qui exécutent ces tâches peuvent également utiliser le même système de lecture pour s'identifier à l'aide d'un badge. Le nom des exécutants peut ainsi être enregistré et stocké dans une base de données associée.

Il est à noter que l'emplacement pour l'instrument chirurgical dans la boîte de rangement s'affiche, par exemple, en surbrillance sur l'interface (50) du système informatique (5).

De façon avantageuse, le système de lecture (4) relié au système informatique (5) peut être utilisé par du personnel de pharmacies pour, par exemple, contrôler la réception de chaque instrument chirurgical, connaître l'historique opératoire, gérer les stocks, faire un suivi de chaque instrument chirurgical, réassortir des commandes d'achat mais aussi par le personnel en charge de la stérilisation dans les établissements de santé pour, par exemple, reconstituer rapidement les boîtes d'instruments médicaux après l'intervention chirurgicale et la décontamination de tous les instruments médicaux. De façon avantageuse, ce système de lecture (4) associé au système informatique (5) et coopérant avec l'instrument chirurgical, tel que la râpe fémorale (1), permet un gain de temps pour la traçabilité ou la récupération d'informations associées à l'instrument chirurgical et permet de gérer efficacement un rappel de l'instrument chirurgical ou un rappel patients. En outre, lors de la lecture du transpondeur (2) de l'instrument chirurgical, il est possible qu'une alarme sonore du système informatique (5) informe l'utilisateur de l'usure de l'instrument chirurgical et par conséquent, de prévoir le rachat d'un nouvel instrument chirurgical.

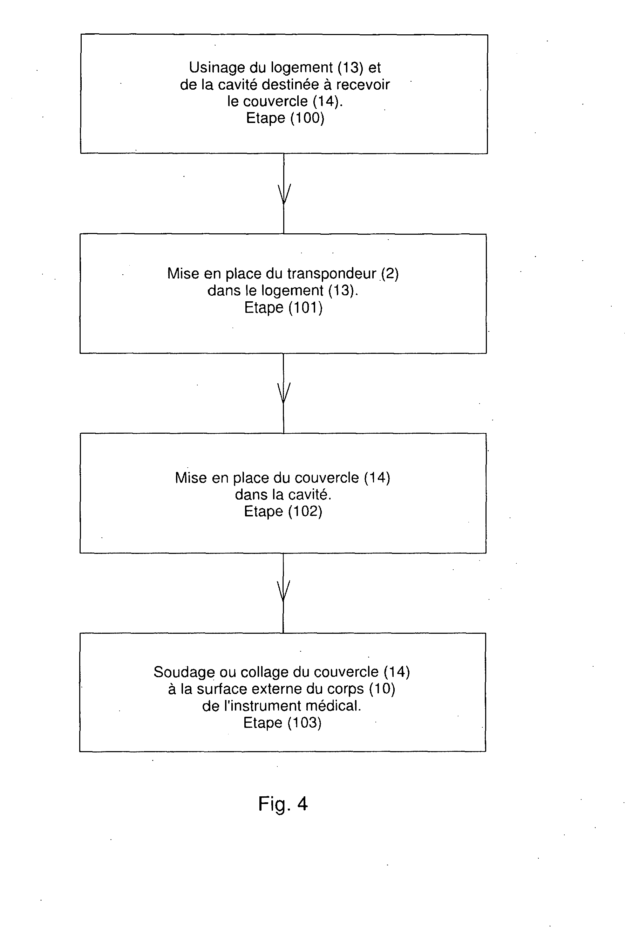

En référence à la figure 4, le procédé d'intégration du transpondeur (2) dans la râpe fémorale (1) consiste à effectuer une opération d'usinage ou alésage (étape 100) pour former le logement (13) de forme sensiblement cylindrique puis la cavité de forme cylindrique destinée à recevoir le couvercle fin (14) du logement (13) de la râpe fémorale (1).

Les alésages pour former le logement (13) et la cavité prévue pour recevoir le couvercle (14) sont réalisés selon un axe (Δ) perpendiculaire à la surface externe du corps (10) de la râpe fémorale (1).

Il est à noter que le diamètre (DI) de l'alésage formant le logement (13) est inférieur au diamètre (Dc) de l'alésage formant la cavité pour le couvercle (14). En outre, l'épaisseur ou la profondeur (el) de l'alésage formant le logement (13) est supérieure à la profondeur (ec) de l'alésage formant la cavité pour le couvercle (14).

En fonction de la zone lisse du corps (10) de la râpe (1) qui est usinée et des dimensions du transpondeur (2), l'alésage pour former le logement (13) doit être réalisé selon une profondeur (el) et un diamètre (DI) définis pour ne pas affecter la râpe fémorale (1) et pour permettre le positionnement du transpondeur (2) sensiblement à proximité de la surface externe du corps (10) de la râpe fémorale (1).

Dans un premier exemple de réalisation, pour un transpondeur (2) de diamètre (Dt) sensiblement égal à 5,6 millimètres et une épaisseur (et) sensiblement égale à 1,7 millimètres, un alésage de diamètre (DI) sensiblement égal à 6,6 millimètres et de profondeur (el) sensiblement égale à 1,9 millimètres est réalisé. Il est à noter que la pointe du foret servant à l'alésage pour former le logement (13) ne doit pas dépasser un diamètre sensiblement égal à 4,5 millimètres pour ne pas fragiliser ou affecter la râpe fémorale (1).

Dans un deuxième exemple de réalisation, pour un transpondeur (2) de diamètre (Dt) sensiblement égal à 7.4 millimètres et une épaisseur (et) sensiblement égale à 2,6 millimètres, un alésage de diamètre (DI) sensiblement égale à 7.5 millimètres et de profondeur (el) sensiblement égale à 2.7 millimètres est réalisé. Il est à noter que la pointe du foret servant à l'alésage pour former le logement (13) ne doit pas dépasser un diamètre sensiblement égal à 6.5 millimètres pour ne pas fragiliser ou affecter la râpe fémorale (1).

Par conséquent, le diamètre (DI) et la profondeur (el) du logement (13) formé par l'opération d'usinage sont respectivement sensiblement supérieurs au diamètre (Dt) et à la profondeur (et) du transpondeur (2).

De façon avantageuse, la faible différence entre les diamètres (Dl, Dt) et les profondeurs (el, et) respectivement du logement (13) et du transpondeur (2) permet au transpondeur (2) d'être caler dans le logement (13) lorsque le couvercle (14) est soudé ou collé au corps (10) de la râpe fémorale (1).

Une fois que le transpondeur (2) est positionné ou calé dans le logement (13) (étape 101), le procédé d'intégration du transpondeur (2) dans la râpe fémorale (1) comporte une étape de positionnement (étape 102) du couvercle (14) dans la cavité prévue pour recevoir ledit couvercle (14) puis une opération de soudage ou de collage (étape 103) du couvercle (14) au corps (10) de la râpe fémorale (1) de façon à ce que la surface externe de la râpe (1) ne présente aucune aspérité saillante.

Un des avantages de l'invention est que l'instrument chirurgical équipé d'un transpondeur (2) permet son identification et sa traçabilité individuelle tout en gardant ces propriétés invasives.

Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit pas être limitée aux détails donnés ci-dessus.

## Revendications

1. Système de lecture (4) destiné à coopérer avec un transpondeur (2) disposé sur un instrument chirurgical de façon à assurer rapidement l'identification de l'instrument chirurgical par approchement du système de lecture (4) au transpondeur (2) de l'instrument chirurgical puis lecture des informations associées à l'instrument chirurgical contenues dans le transpondeur (2) et communication avec un système informatique (5), **caractérisé en ce que** le transpondeur (2) est enfermé dans un logement (13) formé dans l'instrument chirurgical destiné à recevoir à l'intérieur le transpondeur (2), ledit logement (13) étant refermé de façon étanche par un couvercle fin (14), de même manière que l'instrument chirurgical, soudé ou collé (3) au corps (10) de l'instrument chirurgical de façon à ce que la surface externe de l'instrument chirurgical ne présente aucune aspérité saillante, le transpondeur (2) communiquant ou enregistrant par le système de lecture (4) des informations concernant l'historique, les positions de l'instrument chirurgical dans une boîte étant affichées sur un écran du système informatique (5) par affichage des données provenant d'une base de données et associées à la traçabilité.

2. Système de lecture (4) selon la revendication 1, **caractérisé en ce que** l'instrument chirurgical invasif forme une pièce monobloc comprenant le logement (13) formé par une opération d'usinage du corps (10) de l'instrument chirurgical dans une zone lisse ou en retrait du corps (10) qui ne fragilise pas l'instrument chirurgical et ne gêne pas la mise en place d'une poignée.

3. Système de lecture (4) selon les revendications 1 et 2, **caractérisé en ce que** les dimensions du logement (13) et l'épaisseur réduite du couvercle (14) permettent de positionner le transpondeur (2) près de la surface externe du corps (10) de l'instrument chirurgical.

4. Système de lecture (4) selon les revendications 1 à 3, **caractérisé en ce que** le logement (13) et le couvercle (14) sont de forme sensiblement cylindrique.

5. Système de lecture (4) selon les revendications 1 à 4, **caractérisé en ce que** le couvercle (14) du logement (13) de l'instrument chirurgical est réalisé en matériau, présentant des caractéristiques de résistance à la corrosion et de résistance mécanique, tel que l'acier inoxydable ou le titane ou le chrome cobalt ou le polymère, le même matériau étant utilisé pour former le corps (10) de l'instrument chirurgical.

6. Système de lecture (4) selon les revendications 1 à 5, **caractérisé en ce que** le couvercle (14) est assemblé par soudage, tel que le soudage par laser, de façon à assurer une continuité métallurgique entre la surface externe du corps (10) de l'instrument chirurgical et le couvercle (14) et assurer l'étanchéité du logement (13).

7. Système de lecture (4) selon les revendications 1 à 6, **caractérisé en ce que** le couvercle (14) est collé au corps (10) de l'instrument chirurgical de façon à assurer l'étanchéité du logement (13).

8. Système de lecture (4) selon les revendication 1 à 7, **caractérisé en ce que** l'instrument chirurgical est une râpe fémorale (1) comportant un corps (10) allongé, parallèle à l'axe longitudinal du canal de l'os à opérer et une partie supérieure (11) destinée à être utilisée comme moyen de préhension pour manipuler la râpe fémorale (1), le corps (10) de la râpe fémorale (1) comportant une zone supérieure lisse (12a) et se terminant par une forme profilé (12b) en fuseau pour faciliter l'enfoncement de la râpe fémorale (1) dans le canal de l'os, la forme profilé (12b) de la râpe fémorale (1) comportant une denture pour permettre de râper le canal de l'os.

9. Système de lecture (4) selon les revendications 1 à 8, **caractérisé en ce que** le système de lecture est apte à identifier l'utilisateur de l'instrument chirurgical à l'aide d'un badge.

10. Système de lecture (4) selon les revendications 1 à 9, **caractérisé en ce que** le système informatique est un ordinateur personnel.

11. Utilisation du système de lecture (4) selon les revendications 1 à 10, **caractérisée en ce que** le système de lecture est destiné à coopérer avec un instrument chirurgical relié à un système informatique (5) gérant une base de données, l'utilisation comportant :
- une étape de lecture des informations contenues dans le transpondeur (2) calé dans le logement (13) de l'instrument chirurgical au moyen du système de lecture (4) ;
- une étape de transmission des informations lues par le système de lecture (4) à destination du système informatique (5), les informations pouvant être éventuellement transmises par internet au fabricant et/ou établissement où est livré l'instrument chirurgical ;
- une étape de recherche, au moyen d'un logiciel approprié du système informatique (5), dans la base de données du système informatique (5) des données de traçabilité associées à l'instrument chirurgical ; et
- une étape d'affichage sur l'écran (50) du système informatique (5) de l'emplacement pour l'instrument chirurgical dans la boîte de rangement et des données de traçabilité associées à l'instrument chirurgical telles que le numéro de boîte de rangement, le numéro du logement de l'instrument chirurgical dans ladite boîte, le numéro de l'instrument chirurgical et l'historique clinique associé à l'instrument chirurgical ou les données relatives aux patients ou les données opératoires des interventions chirurgicales précédentes.

12. Utilisation du système de lecture (4) selon la revendication 11, **caractérisée en ce que** les données informatiques associées à l'instrument chirurgical et affichées sur l'écran (50) du système informatique (5) peuvent être sélectionnées par l'utilisateur via une interface utilisateur du système informatique (5) et peuvent renseignées sur les conditions d'utilisation, de nettoyage, de stérilisation et de stockage de l'instrument chirurgical.

13. Utilisation du système de lecture selon les revendications 11 et 12 , **caractérisé en ce que** les données de traçabilité associées à l'instrument chirurgical sont aptes à être modifiées au fur et à mesure des utilisations de l'instrument chirurgical, les modifications étant saisies au moyen de l'interface du système informatique (5) puis retransmises via le système de lecture (4) au transpondeur (2) de façon à ce qu'elles soient mémorisées dans le transpondeur (2).

14. Utilisation du système de lecture selon une des revendications 11 à 13, **caractérisée en ce que** les données de traçabilité associées à l'instrument chirurgical peuvent être modifiées au fur et à mesure des utilisations de l'instrument chirurgical, les modifications étant saisies au moyen de l'interface utilisateur du système informatique (5) puis retransmises via le système de lecture (4) au transpondeur (2) de façon à ce qu'elles soient mémorisées dans la mémoire du transpondeur (2).

15. Utilisation du système de lecture selon une des revendications 11 à 14, **caractérisée en ce qu'**une alarme sonore du système informatique (5) informe de l'usure de l'instrument chirurgical et de la prévision du rachat d'un nouvel instrument chirurgical.
